# EUROPÄISCHE PATENTSCHRIFT

(11) **EP 1 304 094 B1**
(45) Veröffentlichungstag und Bekanntmachung des Hinweises auf die Patenterteilung: **30.03.2011**
(21) Anmeldenummer: 02022404.4
(22) Anmeldetag: 04.10.2002
(51) Int. Cl.: A61F 13/20

(54) **Vorrichtung zum Herstellen eines Tampons**
Apparatus for making a tampon
Appariel de fabrication d'un tampon

(30) Priorität: 19.10.2001 DE 10151756
(43) Veröffentlichungstag der Anmeldung: 23.04.2003
(73) Patentinhaber: Ruggli Projects AG, 6332 Hagendorn Cham (CH)
(72) Erfinder: Rolli, Kilian, 5408 Ennetbaden (CH)
(74) Vertreter: Ofner, Clemens

(56) Entgegenhaltungen:
- EP-A- 0 611 562
- CH-A- 613 114
- US-A- 2 425 004

## Beschreibung

Die Erfindung betrifft eine Vorrichtung zum Herstellen eines Tampons aus einem zylindrischen Rohling beliebiger Querschnittsform aus saugfähigem Material, mit einem Presswerkzeug aus. mehreren in bezug auf den zentral darin angeordneten Rohling vor- und zurückbeweglichen Pressbacken, von denen über den Umfang des Rohlings verteilt zumindest ein Teil der Pressbacken mit hervorstehenden und den Rohling beim Pressvorgang mit Längsrillen versehenden Pressschneiden versehen ist, und mit einem Stösselwerkzeug zum axialen Ausschieben des gepressten Vorformlings aus dem Presswerkzeug.

Eine Vorrichtung mit diesen Merkmalen ist aus der EP 1 022 004 A1 bekannt. Mittels vorund zurückbeweglicher Pressbacken, die an ihrem vorderen Ende mit Pressschneiden versehen sind, lassen sich diskrete Bereiche der Umfangsfläche des Tamponrohlings zu einem verhältnismässig hoch verdichteten zentralen Faserkern mit hoher Knickfestigkeit pressen. Die zwischen den Pressschneiden befindlichen Bereiche der Umfangsfläche des Tamponrohlings werden hingegen von im Vergleich zu den Pressschneiden zurückstehenden Pressschultern mit radialem Druck beaufschlagt. Es entstehen auf diese Weise in Längsrichtung verlaufende Rippen, die sich sodann in einem nachgeschalteten Formwerkzeug noch zu einer nahezu geschlossenen Oberfläche verformen lassen, ohne dass sie durch die Faserstruktur negativ beeinflusst wird. Die Überführung in das nachgeschaltete Formwerkzeug erfolgt mittels eines axial in bezug auf die Formkammer der Tamponpresse verfahrbares Stösselwerkzeug. Ein derartiges Stösselwerkzeug ist z. B. aus der DE 93 20 358 U1 bekannt. Nach dem Pressen des Tamponrohlings in der Tamponpresse erfaßt die zylindrische Stirnfläche dieses Stößelwerkzeuges die Stirnfläche des Tamponrohlings, und schiebt diesen nach Überwindung der zwischen Tampon und Preßbacken wirkenden Losbrechkräfte in das nachgeschaltete Formwerkzeug, welches z. B. die Gestalt einer konisch zulaufenden Hülse aufweist. Nachteilig hieran ist, daß wegen der Überwindung der genannten Losbrechkräfte zwischen Tampon und den Preßbacken der Tamponpresse der Stößel eine relativ große Druckkraft auf den Tamponrohling ausüben muß. Dies kann zu unerwünschten Verformungen des Tampons im Bereich von der Stirnfläche führen, bis hin zur Selbsthemmung durch Watte/Fliesmaterial, welches sich beim Ausstoßen um den Außenrand des Stößels herumlegt.

Der Erfindung liegt die **Aufgabe,** zugrunde eine Vorrichtung zum Herstellen eines Tampons zu schaffen, bei der die Überführung des Tampon-Vorformlings aus dem Preßwerkzeug in eine nachgeschaltete Verarbeitungsstufe schonender erfolgt.

Zur **Lösung** dieser Aufgabe wird bei einer Vorrichtung mit den eingangs genannten Merkmalen vorgeschlagen, daß sich die wirksame Stirnfläche des Stößelwerkzeuges aus einer zentralen Druckfläche und radial davon abstehenden Zusatzdruckflächen zusammensetzt, daß der vom äußeren Rand der Zusatzdruckflächen definierte Durchmesser größer ist als der durch den inneren Rand der Preßschneiden definierte minimale Preßdurchmesser, und daß jede Zusatzdruckfläche auf einen der Zwischenräume zwischen zwei aufeinanderfolgenden Preßschneiden axial ausgerichtet ist.

Diese Vorrichtung zeichnet sich durch ein besonders schonendes Überführen des Vorformlings aus dem Preßwerkzeug heraus in eine nachgeordnete Verarbeitungsstufe aus. Die Druckübertragungsfläche, mit der sich das Stößelwerkzeug gegen die Stirnfläche des herauszuschiebenden Vorformlings abstützt, ist relativ groß ausgebildet, so daß die Flächenpressung auf das Fasermaterial des Tampons relativ gering bleibt. Zur Vergrößerung der wirksamen Druckübertragungsfläche setzt sich das Stößelwerkzeug nicht nur aus einer zentralen Druckfläche, sondern zusätzlich aus radial davon abstehenden Zusatzdruckflächen zusammen. Der vom äußeren Rand dieser Zusatzdruckflächen definierte Durchmesser ist größer als der durch den inneren Rand der Preßschneiden definierte minimale Preßdurchmesser, wodurch die Stirnfläche des Stößelwerkzeugs insgesamt eine in etwa sternförmige Gestalt erhält.

Mit einer bevorzugten Ausgestaltung der erfindungsgemäßen Vorrichtung wird vorgeschlagen, daß die Preßbacken außer in die Preßstellung zusätzlich in eine Lüftstellung einstellbar sind, in der der vom inneren Rand der Preßschneiden definierte Durchmesser größer ist als deren minimaler Preßdurchmesser, und daß der Durchmesser der zentralen Druckfläche des Stößelwerkzeuges größer ist als der minimale Preßdurchmesser der Preßschneiden.

Mit einer weiteren Ausgestaltung der erfindungsgemäßen Vorrichtung wird vorgeschlagen, daß die Preßbacken in einer Lüftstellung einstellbar sind, in der der Umfangsabstand zwischen zwei aufeinander folgenden Preßschneiden größer ist als deren Umfangsabstand bei minimal geschlossenen Preßbacken, und daß bei maximal geschlossenen Preßbacken die Breite der Zusatzdruckflächen in Umfangsrichtung größer ist als der Umfangsabstand zwischen zwei aufeinander folgenden Preßschneiden.

Schließlich wird mit einer weiteren Ausgestaltung vorgeschlagen, daß die Zusatzdruckflächen nach außenhin erweitert gestaltet sind. Dies führt zu einer weiteren Flächenvergrößerung der wirksamen Druckfläche zwischen Stößelwerkzeug und Tampon-Vorformling.

Einzelheiten und weitere Vorteile des Gegenstandes der Erfindung ergeben sich aus der nachfolgenden Beschreibung eines bevorzugten Ausführungsbeispieles. In den Zeichnungen zeigen im Einzelnen:
- Figur 1: eine schematische Ansicht einer Vorrichtung zum Pressen eines Tampons mit Preßwerkzeugen in einer völlig geöffneten Stellung;
- Figur 2: eine schematische Ansicht der Vorrichtung nach Figur 1 in einer geringfügig geschlossenen Haltestellung;
- Figur 3: eine schematische Ansicht der Vorrichtung nach Figur 1 in maximal geschlossener Preßstellung;
- Figur 4: eine schematische Ansicht der Vorrichtung nach Figur 1 in einer dem Preßvorgang folgenden Lüftstellung;
- Figur 5: einen Längsschnitt durch die Vorrichtung zum Pressen eines Tampons mit einem axial vor dem Preßwerkzeug angeordneten Stößelwerkzeug;
- Figur 6: in einer Detaildarstellung der Figur 3 die Preßbacken des Preßwerkzeuges in maximal geschlossener Preßstellung und
- Figur 7: die Preßbacken nach Figur 6 in Lüftstellung einschließlich des darin axial eingefahrenen Stößelwerkzeuges.

Das auf der Zeichnung dargestellte Preßwerkzeug zum Pressen eines Tampons weist insgesamt acht gleich ausgebildete Einzelwerkzeuge auf, die gleichmäßig verteilt über den Umfang eines zu pressenden Tamponrohlings 1 angeordnet sind. Diese Anzahl kann auch von acht abweichen. Bestandteile jedes einzelnen Preßwerkzeuges sind eine Preßbacke 2 mit einer daran zusätzlich angeformten Preßschneide 3. Die Preßbacken 2 mit den Preßschneiden 3 lassen sich in einer im wesentlichen radialen Schwenkbewegung auf den Tamponrohling 1 zu bewegen, um diesen durch in erster Linie radiale Kräfte zu pressen und hierbei einen sog. Vorformling zu erzeugen. Die an den Preßbacken 2 ausgebildeten Preßschneiden 3 führen hierbei zur Bildung von Längsrillen in dem Tampon. Nach dem Abschluß des Preßverfahrens setzt sich der Vorformling aus einem zentralen Tamponkern und sich von dem Tamponkern radial nach außen erstreckenden Längsrippen zusammen, wobei die Längsrippen durch die durch die Preßschneiden 3 erzeugten Längsrillen voneinander getrennt sind.

Jede der Preßbacken 2 ist an dem freien Ende eines Preßhebels 4 befestigt und ist im wesentlichen rechtwinklig zu dem Preßhebel 4 ausgerichtet. Diese Befestigung kann einstückig sein, alternativ lassen sich die einzelnen Preßbacken 2 auch auswechselbar am Ende der Preßhebel 4 befestigen, um so die Preßbacken gegen andere Preßbacken austauschen zu können.

Die vorzugsweise gekrümmt gestalteten Preßhebel 4 sind an ihrem anderen Ende auf einer ein Gelenk bildenden Lagerachse 5 gelagert. Die Lagerachsen 5 sämtlicher Preßhebel 4 befinden sich auf einem feststehenden Stützring 6 oder einem anderen feststehenden Bauteil der Tamponpresse. Hierbei sind alle Lagerachsen 5 auf einem gemeinsamen, gedachten Kreis 7 fixiert, so daß die Lage aller Lagerachsen 5 der Preßhebel zueinander unveränderlich ist.

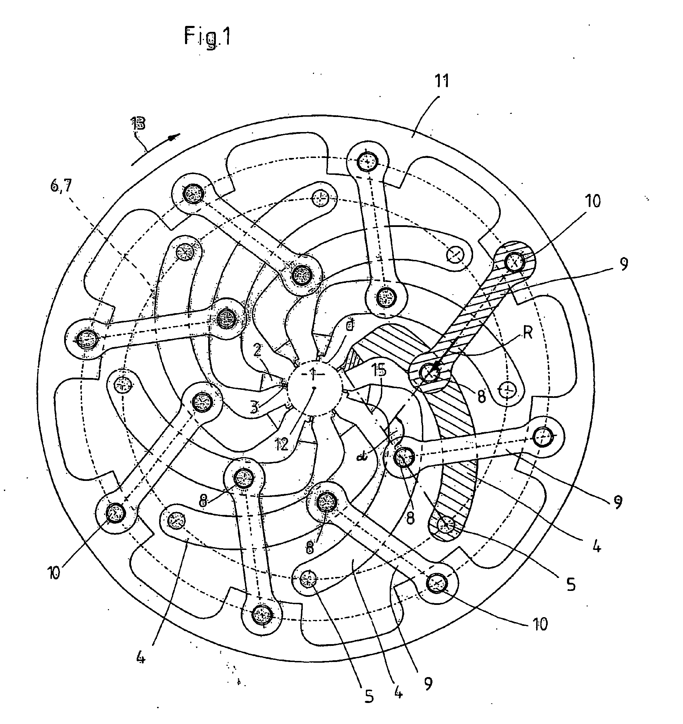

Zur Verdeutlichung ist in Figur 1 einer der insgesamt acht Preßhebel 4 durch eine entsprechende Schraffierung hervorgehoben.

Die Preßhebel 4 sind mit daran befestigten Drehzapfen 8 versehen, über die die Preßhebel gelenkig mit Druckstäben 9 verbunden sind. Vorzugsweise befindet sich auf jeder Seite der Preßhebel 4 jeweils ein Druckstab 9, um so eine querkraftfreie Krafteinleitung von den Druckstäben 9 auf den Preßhebel zu ermöglichen. Der Drehzapfen 8 bildet ein Gelenk, welches, wie Figur 1 erkennen läßt, näher an der Preßbacke 2 denn an der Lagerachse 5 des Preßhebels 4 sitzt. Beim Ausführungsbeispiel befindet sich das durch den Drehzapfen 8 gebildete Gelenk in etwa im zweiten Drittel der Länge des gebogenen Preßhebels 4, von dessen Lagerachse 5 aus betrachtet.

Auch das andere Ende des Druckstabes 9 ist gelenkig gelagert und zwar an einem Drehzapfen 10 eines ringförmigen Antriebselementes 11. An dem ringförmigen Antriebselement 11 sind in entsprechender Weise, gleichmäßig über den Umfang verteilt, auch die Drehzapfen 10 für alle übrigen Druckstäbe 9 angeordnet. Das ringförmige Antriebselement 11 ist um eine Drehachse 12 gelagert, die mit der Mittelachse des Tampons 1 zusammenfällt, so daß die beschriebene Tamponpresse insgesamt völlig symmetrisch aufgebaut ist. Insbesondere bewegt sich das ringförmige Antriebselement 11 konzentrisch nicht nur zu dem Tampon 1, sondern auch zu dem Kreis 7, auf dem die feststehenden Lagerachsen 5 für die Preßhebel 4 angeordnet sind.

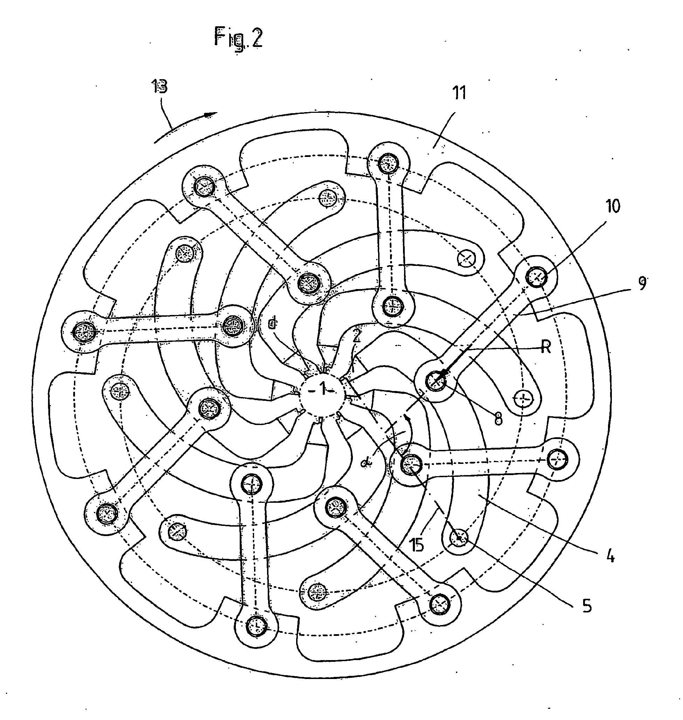

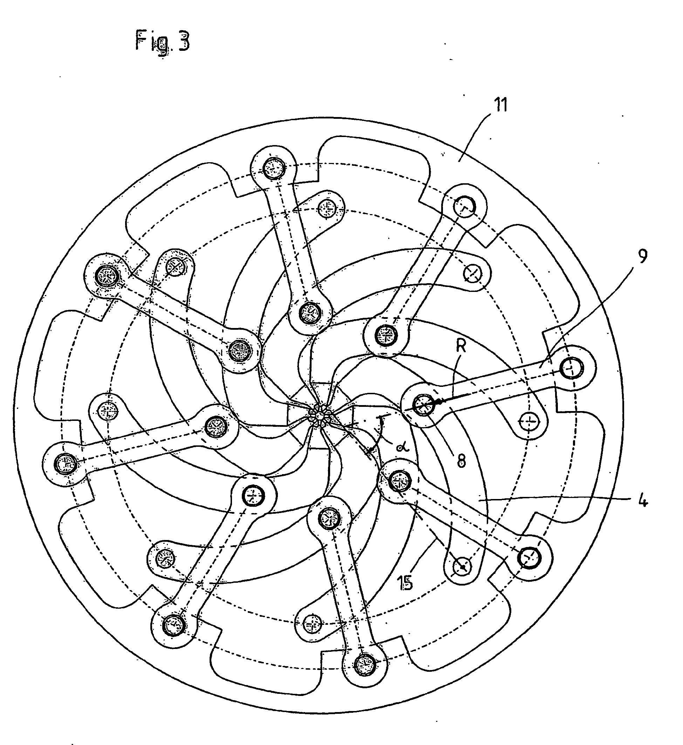

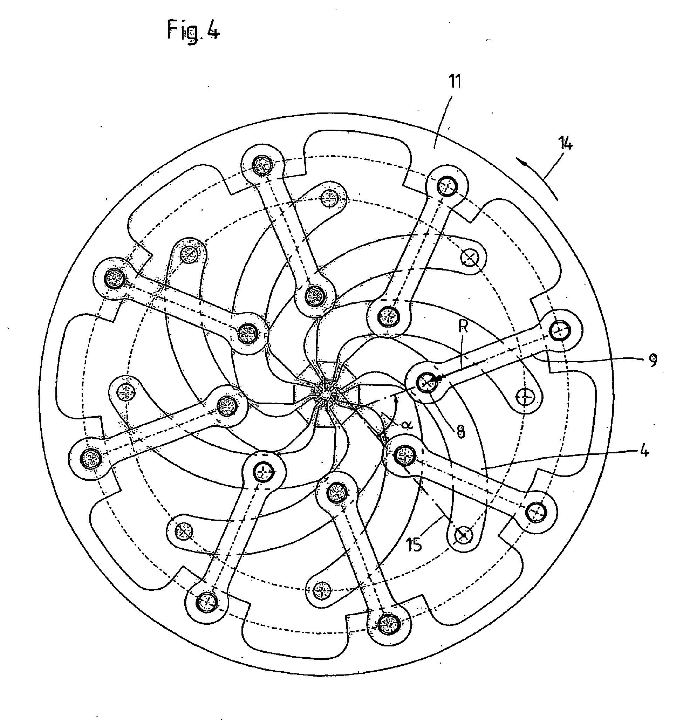

Figur 1 zeigt die Tamponpresse in geöffnetem Zustand und damit in jenem Zustand, in welchem der Tamponrohling 1 axial in den von den geöffneten Preßbacken gebildeten zylindrischen Raum eingeschoben wird. Figur 2 zeigt eine demgegenüber bereits mehr geschlossene Stellung der Preßbacken zur Ausübung einer Haltefunktion auf den Tamponrohling. Figur 3 zeigt die Tamponpresse im Zustand des maximalen Preßweges und damit auch des maximalen Preßdruckes innerhalb des Tampons. Die Preßbacken 2 sind weitestmöglich geschlossen. Da es nun in dem Zustand gemäß Figur 3 nahezu unmöglich ist, den vorgepreßten Vorformling wieder axial aus der Tamponpresse hinauszuschieben, erfolgt in einer letzten Stufe, die in Figur 4 dargestellt ist, ein wieder teilweises Öffnen bzw. Lüften der Preßbacken 2. In diesem Zustand kann der gebildete Vorformling mittels eines Stößels axial aus dem durch die Preßbacken gebildeten Formraum ausgeschoben werden.

Die in den Figuren 1 bis 4 schrittweise dargestellte Bewegung der Tamponpresse wird ausschließlich durch Verdrehen des ringförmigen Antriebselementes 11 in Richtung des in den Figuren 1 und 2 eingezeichneten Pfeiles 13 erzeugt. Der Pfeil 14 in Figur 4 symbolisiert die Öffnungsbewegung des ringförmigen Antriebselements 11 zum Zwecke des Lüftens der Tamponpresse. In der gelüfteten Stellung gemäß Figur 4 läßt sich der Vorformling mittels des Stößels axial ausstoßen und in ein nachgeschaltetes Formwerkzeug einführen, in dem er seine Endform erhält. Dieses nachgeschaltete Formwerkzeug ist regelmäßig als sich konisch verjüngende Hülse ausgebildet, in der der Vorformling auf seinen endgültigen Durchmesser verformt wird.

In den Figuren 1 bis 4 ist jeweils der Winkel α eingezeichnet, welchen der als Übertragungselement dienende Druckstab 9 in bezug auf die gedachte Verbindungslinie 15 zwischen Lagerachse 5 und Preßbacke 2 einnimmt. In den Stellungen nach den Figuren 1 und 2 ist dieser Winkel α etwas größer als 90°, in den Stellungen nach den Figuren 3 und 4 etwas kleiner als 90°. Dies bedeutet, daß zumindest zu einem Zeitpunkt des Preßvorgangs, der z. B. zwischen den Stellungen nach den Figuren 2 und 3 anzusiedeln ist, die Richtung R der von dem Druckstab 9 auf den Preßhebel 4 ausgeübten Krafteinleitung im wesentlichen quer, d. h. rechtwinklig zu der gedachten Verbindungslinie 15 zwischen Lagerachse 5 und Preßbacke 2 erfolgt. In mindestens einer Schwenklage des Preßhebels 4 erstreckt sich daher die Kraftübertragungsrichtung R des als Übertragungselementes dienenden Druckstabes 9 im wesentlichen quer zu dieser gedachten Verbindungslinie 15. In diesem Winkelbereich, der teilweise geringfügig kleiner und teilweise geringfügig größer als 90° ist, ist die Kraftübertragung optimal, d. h. es lassen sich bei moderater Antriebsleistung für das ringförmige Antriebselement 11 sehr hohe Preßkräfte auf den Preßhebel 4 und damit auf die dort rechtwinklig angeordnete Preßbacke 2 erzeugen. Im Zustand nach Fig. 3 ist die Richtung R nahezu direkt auf den Tampon 1 ausgerichtet.

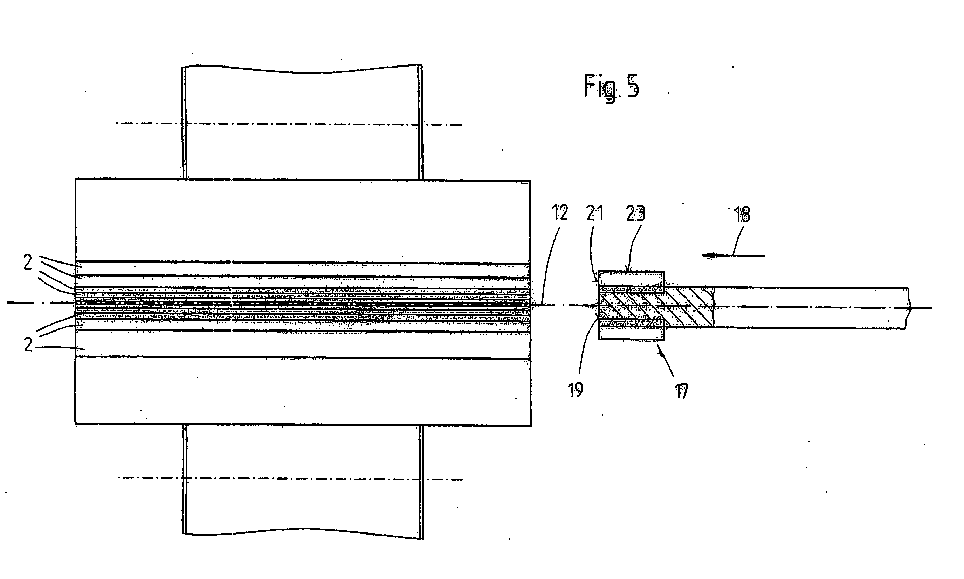

Die Figur 5 zeigt das gesamte Preßwerkzeug in einem stark vereinfachten Längsschnitt, wobei sich axial vor dem Preßwerkzeug und in Fluchtung von dessen Drehachse 12 ein Stößelwerkzeug 17 befindet. Das Stößelwerkzeug 17 ist in der mit Bezugszeichen 18 versehenen Richtung axial in den zentralen Formraum des aus den Preßbacken 2 gebildeten Preßwerkzeuges einfahrbar. Hierbei nimmt das Stößelwerkzeug 17 den Vorformling mit, und fördert diesen in ein der Vorrichtung nachgeschaltetes Formwerkzeug, bei dem es sich z. B. um eine zu ihrem Ende hin konisch zulaufende Hülse handeln kann, in welcher der Vorformling seine endgültige Gestalt erhält.

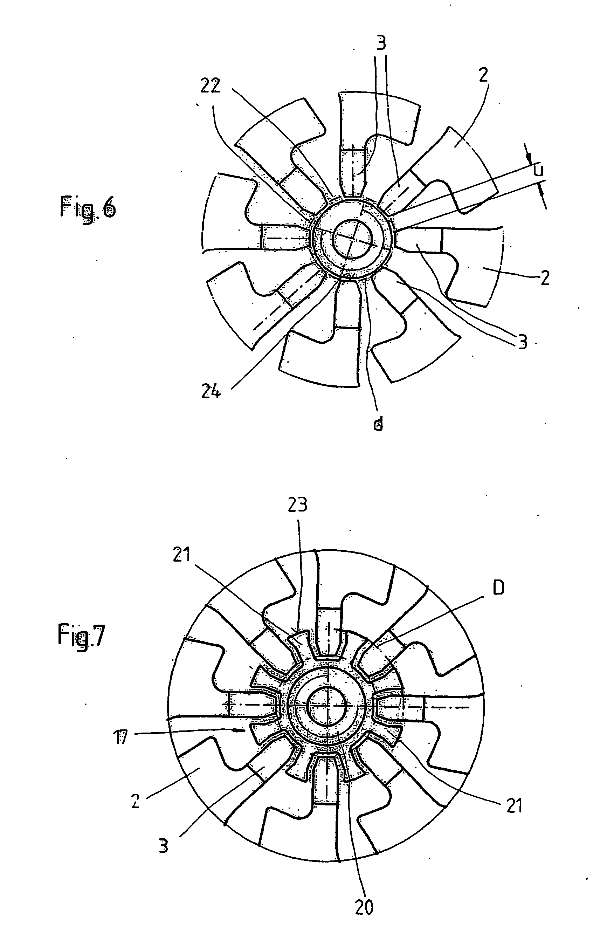

Die den Vorformling erfassende vordere Stirnfläche 19 des Stößelwerkzeugs 17 weist eine sternförmige Gestalt auf, was nachfolgend unter Bezugnahme auf die Figuren 6 und 7 näher erläutert wird.

Figur 6 zeigt gegenüber Figur 3 stark vergrößert die einzelnen Preßbacken 2 mit den Preßschneiden 3 während des eigentlichen Preßvorgangs, d. h. bei maximal geschlossenen Preßbacken. Der Vorformling ist in diesem Zustand sehr stark verdichtet, weshalb es nicht möglich wäre, den Vorformling in diesem Zustand aus den vollständig geschlossenen Preßbacken axial herauszustoßen. Aus diesem Grund erfolgt das Herausstoßen mittels des Stößelwerkzeuges in der auch in Figur 4 dargestellten Lüftstellung der Tamponpresse. Diese Lüftstellung ist auch in Figur 7 dargestellt, und zwar gegenüber Figur 4 stark vergrößert und unter zusätzlicher Einzeichnung der Stirnfläche 19 des Stößelwerkzeuges 17. Zu erkennen ist, daß die Stirnfläche 19 sich aus einer zentralen, kreisförmigen Druckfläche 20 und radial davon abstehenden Zusatzdruckflächen 21 zusammensetzt. Die Anzahl der Zusatzdruckflächen 21 ist gleich der Anzahl der Preßschneiden 3, außerdem sind, in axialer Richtung betrachtet, die Zusatzdruckflächen 21 jeweils auf einen Zwischenraum 22 (Fig. 6) zwischen zwei aufeinanderfolgenden Preßschneiden 3 ausgerichtet. Die Zusatzdruckflächen 21 des Stößelwerkzeuges 17 "passen" also genau in diese Zwischenräume 22, wenn die Tamponpresse ihren gelüfteten Zustand aufweist. Der vom äußeren Rand 23 der Zusatzdruckflächen 21 definierte Durchmesser D ist größer als der durch den inneren Rand 24 (Fig. 6) der Preßschneiden 3 definierte Preßdurchmesser d, wobei dies sowohl in bezug auf den Preßdurchmesser d bei geschlossener Tamponpresse, als auch in bezug auf den Preßdurchmesser d in der Lüftstellung der Tamponpresse gilt. In der Lüftstellung ist der vom inneren Rand 24 der Preßschneiden 3 definierte Durchmesser D größer als deren minimaler Preßdurchmesser. In dieser Lüftstellung ist auch der Durchmesser der zentralen Druckfläche 20 des Stößels größer als der in Figur 6 eingezeichnete minimale Preßdurchmesser d der Preßschneiden 3.

Ferner ist, wie ein Vergleich der Figuren 6 und 7 zeigt, in dieser Lüftstellung der Umfangsabstand u zwischen zwei aufeinander folgenden Preßschneiden 3 größer als deren Umfangsabstand u bei maximal geschlossenen Preßbacken. Bei maximal geschlossenen Preßbacken entsprechend Figur 6 ist die Breite der Zusatzdruckflächen 21 in Umfangsrichtung betrachtet größer als die Breite des Zwischenraumes 22 zwischen zwei aufeinander folgenden Preßschneiden 3. Bei geschlossener Tamponpresse entsprechend Figur 6 wäre es dem Stößelwerkzeug 17 daher unmöglich, in den Formraum einzufahren. Dies ist vielmehr erst bei gelüfteter Tamponpresse entsprechend Figur 7 möglich.

Gemäß Figur 7 sind die Zusatzdruckflächen 21 zu ihrem äußeren Rand 23 hin erweitert gestaltet. Dies führt zu einer nochmals vergrößerten Stirnfläche des Stößelwerkzeuges und damit zu einer Reduzierung der Druckeinwirkung auf den Vorformling beim Ausschieben und insbesondere bei der anfänglichen Überwindung der Haftreibung zwischen Tampon und Preßschneiden.

### Bezugszeichenliste:

- 1: Tampon
- 2: Preßbacken
- 3: Preßschneide
- 4: Preßhebel
- 5: Lagerachse, Gelenk
- 6: Stüztring
- 7: Kreis
- 8: Drehzapfen
- 9: Druckstab
- 10: Drehzapfen
- 11: Antriebselement
- 12: Drehachse
- 13: Pfeil
- 14: Pfeil
- 15: Verbindungslinie
- 16: Mittellinie
- 17: Stößelwerkzeug
- 18: Richtung
- 19: Stirnfläche
- 20: zentrale Druckfläche
- 21: Zusatzdruckfläche
- 22: Zwischenraum
- 23: äußerer Rand
- 24: innerer Rand
- α: Winkel
- d: Preßdurchmesser
- D: Durchmesser
- R: Richtung der Krafteinleitung
- u: Abstand in Umfangsrichtung

## Patentansprüche

1. Vorrichtung zum Herstellen eines Tampons aus einem zylindrischen Rohling beliebiger Querschnittsform aus saugfähigem Material, mit einem Presswerkzeug aus mehreren in bezug auf den zentral darin angeordneten Rohling (1) vor- und zurückbeweglichen Pressbacken (2), von denen über den Umfang des Rohlings (1) verteilt zumindest ein Teil der Pressbacken (2) mit hervorstehenden und den Rohling beim Pressvorgang mit Längsrillen versehenden Pressschneiden (3) versehen ist, und mit einem Stösselwerkzeug (17) zum axialen Ausschieben des gepressten Vorformlings aus dem Presswerkzeug, **dadurch gekennzeichnet, dass** sich die wirksame Stirnfläche (19) des Stösselwerkzeuges (17) aus einer zentralen Druckfläche (20) und radial davon abstehenden Zusatzdruckflächen (21) zusammensetzt, dass der vom äusseren Rand (23) der Zusatzdruckflächen (21) definierte Durchmesser (D) grösser ist als der durch den inneren Rand (24) der Pressschneiden (3) definierte minimale Pressdurchmesser (d), und dass jede Zusatzdruckfläche (21) auf einen der Zwischenräume (22) zwischen zwei aufeinander folgenden Pressschneiden (3) axial ausgerichtet ist.

2. Vorrichtung nach Anspruch 1, **dadurch gekennzeichnet, dass** die Pressbacken (2) in eine Lüftstellung einstellbar sind, in der der vom inneren Rand (24) der Pressschneiden (3) definierte Durchmesser (D) grösser ist als deren minimaler Pressdurchmesser, und dass der Durchmesser der zentralen Druckfläche (20) des Stösselwerkzeuges (17) grösser ist als der minimale Pressdurchmesser der Pressschneiden (3).

3. Vorrichtung nach Anspruch 1 oder Anspruch 2, **dadurch gekennzeichnet, dass** die Pressbacken (2) in eine Lüftstellung einstellbar sind, in der der Umfangsabstand zwischen zwei aufeinander folgenden Pressschneiden (3) grösser ist als deren Umfangsabstand (u) bei maximal geschlossenen Pressbacken (2), und dass bei maximal geschlossenen Pressbacken (2) die Breite der Zusatzdruckflächen (21) in Umfangsrichtung grösser ist, als der Umfangsabstand (u) zwischen zwei aufeinander folgenden Pressschneiden (3).

4. Vorrichtung nach einem der Ansprüche 1 bis 3, **dadurch gekennzeichnet, dass** die Zusatzdruckflächen (21) nach aussenhin erweitert gestaltet sind.

## Claims

1. Device for producing a tampon from a cylindrical blank of absorbent material of any cross-sectional shape, with a pressing mould comprising several pressing jaws (2) which can be moved forwards and backwards relative to the blank (1) disposed centrally therein, of which at least some of the pressing jaws (2) distributed around the circumference of the blank (1) are provided with protruding pressing edges (3) and impart longitudinal grooves to the blank during the pressing process, and with a ram (17) for pushing the pressed preform axially out of the pressing mould, **characterised in that** the active end face (19) of the ram (17) is made up of a central pressing surface (20) and peripheral pressing surfaces (21) protruding radially out from it, and the diameter (D) defined by the outer edge (23) of the peripheral pressing surfaces (21) is bigger than the minimum pressing diameter (d) defined by the inner edge (24) of the pressing edges (3), and each peripheral pressing surface (21) is axially oriented towards one of the gaps (22) between two consecutive pressing edges (3).

2. Device as claimed in claim 1, **characterised in that** the pressing jaws (2) can be set in a venting position in which the diameter (D) defined by the inner edge (24) of the pressing edges (3) is bigger than their minimum pressing diameter, and the diameter of the central pressing surface (20) of the ram (17) is bigger than the minimum pressing diameter of the pressing edges (3).

3. Device as claimed in claim 1 or claim 2, **characterised in that** the pressing jaws (2) can be set in a venting position in which the circumferential distance between two consecutive pressing edges (3) is bigger than their circumferential distance (u) when the pressing jaws (2) are closed to the maximum, and when the pressing jaws (2) are closed to the maximum the width of the peripheral pressing surfaces (21) in the circumferential direction is bigger than the circumferential distance (u) between two consecutive pressing edges (3).

4. Device as claimed in one of claims 1 to 3, **characterised in that** the peripheral pressing surfaces (21) are designed so that they can be widened towards the outside.

## Revendications

1. Dispositif destiné à la fabrication d'un tampon à partir d'une ébauche cylindrique en matériau absorbant ayant une forme de section quelconque, comportant un outil de pressage formé par plusieurs mâchoires de pressage (2), qui sont mobiles vers l'avant et l'arrière par rapport à l'ébauche (1) disposée au centre dudit outil de pressage et parmi lesquelles au moins une partie des mâchoires de pressage (2), réparties sur le pourtour de l'ébauche (1), sont munies d'arêtes de coupe (3) saillantes, destinées à réaliser des cannelures longitudinales dans l'ébauche pendant le processus de pressage, et comportant un outil poussoir (17) destiné à pousser la préforme comprimée hors de la presse dans la direction axiale, **caractérisé en ce que** la face frontale (19) active de l'outil poussoir (17) est formée par une surface de pression (20) centrale et des surfaces de pression supplémentaires (21) radialement en saillie sur cette dernière, **en ce que** le diamètre (D), défini par le bord extérieur (23) des surfaces de pression supplémentaires (21), est supérieur au diamètre de pressage (d) minimum, défini par le bord intérieur (24) des arêtes de coupe (3), et **en ce que** chaque surface de pression supplémentaire (21) est orientée axialement vers l'un des espaces intermédiaires (22) entre deux arêtes de coupe (3) consécutives.

2. Dispositif selon la revendication 1, **caractérisé en ce que** les mâchoires de pressage (2) peuvent être réglées dans une position desserrée, dans laquelle le diamètre (D), défini par le bord intérieur (24) des arêtes de coupe (3), est supérieur au diamètre de pressage minimum de celles-ci, et **en ce que** le diamètre de la surface de pression (20) centrale de l'outil poussoir (17) est supérieur au diamètre de pressage minimum des arêtes de coupe (3).

3. Dispositif selon la revendication 1 ou la revendication 2, **caractérisé en ce que** les mâchoires de pressage (2) peuvent être réglées dans une position desserrée, dans laquelle la distance circonférentielle entre deux arêtes de coupe (3) consécutives est supérieure à la distance circonférentielle (u) de celles-ci lorsque les mâchoires de pressage (2) sont fermées au maximum, et **en ce que**, lorsque les mâchoires de pressage (2) sont fermées au maximum, la largeur des surfaces de pression supplémentaires (21) dans la direction circonférentielle est supérieure à la distance circonférentielle (u) entre deux arêtes de coupe (3) consécutives.

4. Dispositif selon l'une quelconque des revendications 1 à 3, **caractérisé en ce que** les surfaces de pression supplémentaires (21) sont conçues en s'élargissant vers l'extérieur.
